# EUROPÄISCHE PATENTANMELDUNG

(11) **EP 1 923 044 A1**
(43) Veröffentlichungstag der Anmeldung: **21.05.2008**
(21) Anmeldenummer: 07021973.8
(22) Anmeldetag: 13.11.2007
(51) Int. Cl.: A61K 8/60, A61K 8/73, A61K 8/04, A61K 8/06, A61Q 17/04, A61Q 19/00

(54) **Kosmetische Zubereitung und deren Konzentrat mit verbesserten Eigenschaften**

(30) Priorität: 14.11.2006 DE 102006053894
(71) Anmelder: Beiersdorf AG, 20253 Hamburg (DE)
(72) Erfinder: Weyland, Silke, 67551 Worms (DE); Hoffmann, Nils, 22303 Hamburg (DE)

(57) **Zusammenfassung**

Die Erfindung umfasst O/W Emulsionen oder emulgatorfreie Hydrodispersionen umfassend ein oder mehrere Celluloseether, ein oder mehrere aus Stärke gewonnene wasserlösliche Kohlenhydrate und ein oder mehrere nicht wassedöslich modifizierte Stärke.

Die Kombination der drei Komponenten führt zu einer verbesserten Trocknungseigenschaft der sie enthaltenden Emulsionen bzw, Hydrodispersionen und zu einem daraus herstellbaren oder anwendbaren kosmetischen Endprodukt, dass gegenüber anderen Emulsionskonzentraten ein angenehmeres Hautgefühl bereitet.

## Beschreibung

Die Erfindung umfasst kosmetische Zubereitungen, insbesondere O/W Emulsionen oder emulgatorfreie Hydrodispersionen, umfassend ein oder mehrere Celluloseether, ein oder mehrere aus Stärke gewonnene wasserlösliche Kohlenhydrate und ein oder mehrere nicht wasserlösliche modifizierte Stärken.
Die Kombination der drei Komponenten führt zu einer verbesserten Trocknungseigenschaft der sie enthaltenden Emulsionen bzw. Hydrodispersionen und zu einem daraus herstellbaren oder anwendbaren kosmetischen Endprodukt, dass gegenüber anderen Emulsionskonzentraten ein angenehmeres Hautgefühl bereitet.

Hautpflegeprodukte bestehen in der Regel aus Emulsionen. Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußarlich und mit bloßem Auge betrachtet erscheinen Emulsionen homogen.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z.B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Hydroxypropylmethylcellulose und Maltodextrine sind bekannte Trägermaterialien für die Sprühtrocknung (s. z.B. K.L. Christensen et al, Technical optimisation of redispersible dry emulsions, International Journal of Pharmaceutics: C.Turchiuli; Oil encapsulation by spray drying and fluidised bed agglomeration, Innovative Food Science & Emerging Technologies).
Auch modifizierte Stärken werden beispielsweise in der Patentanmeldung WO 0105926 A1 als Trägermaterialien zur Sprühtrocknung eingesetzt.
Diese Schriften beschreiben dabei vornehmlich die Herstellung von Pulvern die entweder oral aufgenommen werden, zur Verkapselung von Parfümstoffen oder als Pulverzusatz in Waschmitteln dienen. Diese Schriften haben gemeinsam, dass die redispergierte Emulsion nicht als Kosmetikum, zum Auftragen auf die Haut, bestimmt sind und die Sensorik, ein für die Kosmetik entscheidender Aspekt, völlig außer Acht gelassen wird

Aus diesem Grund kann der Anteil an Trägermaterialien bis zu 50 Gew% der Emulsion betragen. Solch hohe Mengen an Trägermaterialien sind in einer kosmetischen Emulsion sensorisch jedoch nicht tragbar.

Eine Beschreibung getrockneter Emulsionen, die sich durch die Zugabe von Wasser wieder in eine Creme überführen lassen, ist z.B. in WO 2006034982 A1, DE 102004 025357 A1 und DE 10254335 A1 wiedergegeben.
Ferner werden sprühgetrocknete Emulsionen zum Auftrag auf die Haut in den Schriften EP 664112 B1 und EP 1141097 B1 beschrieben,

Nachteilig insbesondere bei sprühgetrockneten Emulsionen ist es, dass die Ausbeute aufgrund von Verklebung und Anhaftung an den Sprühtrocknungsvorrichtungen häufig gering ist.

Wünschenswert ist es daher eine kosmetische Zubereitung zur Verfügung zu stellen, die bei Trocknung eine höhere Ausbeute an Konzentrat ermöglicht und damit eine höhere Wirtschaftlichkeit bei der Herstellung getrockneter kosmetischer Zubereitungen ergibt.

Die Erfindung umfasst eine Zubereitung, bevorzugt auf Basis einer O/W Emulsion oder auf Basis einer emulgatorfreien Hydrodispersion, umfassend ein oder mehrere Celluloseether, ein oder mehrere aus Stärke gewonnene wasserlösliche Kohlenhydrate und ein oder mehrere nicht wasserlösliche modifizierte Stärken,

Ebenso umfasst die vorliegende Erfindung ein Konzentrat erhältlich durch Trocknung einer Zubereitung mit den zuvor dargelegten Bestandteilen bzw. Zusammensetzungen. Bevorzugt erfolgt die Trocknung durch Sprühtrocknung, Gefriertrocknung, Kanaltrocknung und/oder Walzentrocknung, insbesondere durch Sprühtrocknung.
Konzentrat bedeutet dabei eine wasserfreie Zubereitung bzw. eine Zubereitung mit einem Wassergehalt von ungefähr maximal 8 Gew,-%, bezogen auf das Gesamtgewicht der getrockneten Zubereitung.

Als Celluloseether werden erfindungsgemäß bevorzugt Hydroxypropylmethylcellulosen, Methylhydroxyethylcellulosen, Methylhydroxypropylcellulosen, Methylhydroxybutylcellulosen, Ethylhydroxyethylecellulosen, Carboxymethylhydroxyethylcellulosen, Carboxymethylcellulosen, Methylcellulosen, Hydroxyethylcellulosen, Carboxymethylhydroxyethylcellulosen, Ethylcellulosen, Ethylhydroxyethylcellulosen und/oder Hydroxypropylcellulosen, insbesondere Hydroxypropylmethylcellulose (HPMC), gewählt.
Hydroxypropylmethylcellulose ist beispielsweise unter Pharmacoat 606 der Firma ShinEtsu erhältlich.

Als aus Stärke gewonnene wasserlösliche Kohlenhydrate werden erfindungsgemäß insbesondere Stärkehydrolysate verstanden und gewählt und dabei insbesondere Maltose, Maltit, Glucasesirup, Maltodextrine, Cyclodextrine, Dextrose, Trockenglucosesirup, Sorbit und/oder Fructose, insbesondere Maltodextrine, gewählt. Bevorzugt werden als Maltodextrine mit einem Dextrose-Äquivalent von 19 oder 12, insbesondere 12. Bezeichnet werden diese Maltodextrine mit z.B. MD 12.
MD12 steht für Maltodextrin 12. Das wiederum bedeutet, dass dieses Maltodextrin ein Dextrose-Äquivalent von 12 aufweist. Das Dextrose-Äquivalent beschreibt die Zunahme der Reduktionsfähigkeit einer Stärke-Lösung mit fortschreitender Hydrolyse. Anhand der Dextrose Äquivalente lassen sich die verschiedenen Maltodextrine unterscheiden. Native Stärke hat den Wert DE = 0, nach vollständiger Hydrolyse zu Glucose beträgt der Wert theoretisch DE = 100, und eine vollständige Spaltung zu einem Maltosesirup führt zum Wert DE = 50.
Die Maltodextrine MD 12 und MD 19 wurden getestet, wobei Maltodextrin 12 eindeutig bevorzugt wird.
Maltodextrine sind beispielsweise als Glucidex IT12 der Firma Roquette erhältlich.

Als nicht wasserlösliche modifizierte Stärken werden erfindungsgemäß Stärkeester und/oder -ether, insbesondere Succinate, Xanthogenate, Acetate, Phosphate, Sulfate und/oder Nitrate, insbesondere Aluminium-Stärke-Octenylsuccinat, gewählt.
Aluminium Starch Octenyl Succinat ist beispielsweise erhältlich als Fluidamid DF-12 der Firma MultiChem Inc. oder als Dry Flo-PC der Firma National Starch erhältlich.
Der Begriff "nicht wasserlöslich" umfasst demnach Stärkeverbindungen, die keine oder zumindest nur geringe Wasserlöslichkeit aufweisen.

Durch die Kombination der drei Trägermaterialien, Celluloseether, aus Stärke gewonnene wasserlösliche Kohlenhydrate und nicht wasserlösliche modifizierte Stärken, konnte eine Zubereitung, insbesondere Emulsion bzw. Hydrodispersion, mit guten Sprühtrocknungseigenschaften sowie einem angenehmen Hautgefühl entwickelt werden.

Das verbesserte Hautgefühl der erfindungsgemäßen Zubereitung bzw. dessen Konzentrat zeigt sich in erster Linie in einer reduzierten Klebrigkeit der Zubereitung bzw. der sich bildenden Emulsion auf der Haut.
Ohne die erfindungsgemäße Dreierkombination und im Vergleich der Einzelbestandteile alleine, vor allem aber bei HPMC alleine, hinterließ die Emulsion ein stark klebriges Hautgefühl.

Als wesentliche Verbesserung der erfindungsgemäßen Kombination kann zum einen der Ertrag des Endproduktes bei der Trocknungsherstellung durch die Kombination der drei Komponenten erhöht werden, da wesentlich weniger Produkt an den Wänden des Sprühturms kleben bleibt. Des weiteren ist das getrocknete Pulver "fließfähiger".
Emulsionen, die beispielsweise nur mit HPMC hergestellt wurden, zeigen eine sehr klebrige Sensorik.
Durch die erfindungsgemäße Kombination, insbesondere HPMC mit Maltodextrin, kann der Anteil an HPMC gesenkt werden und das Produkt klebt nicht mehr so stark.
Durch den Zusatz von Al-Starch-Octenylsuccinat erhält die Emulsion zudem eine leicht pudrige Sensorik, das Kleben verschwindet immer mehr und die Emulsion lässt sich besser trocknen. Ein Kleben am Sprühturm während der Trocknung zum Konzentrat wird vermindert bzw. vermieden.

Die Kombination aus ein oder mehreren Celluloseethern, insbesondere HPMC, ein oder mehreren aus Stärke gewonnenen wasserlöslichen Kohlenhydraten, insbesondere Maltodextrine, und ein oder mehreren nicht wasserlöslichen modifizierten Stärken, insbesondere Aluminium-Stärke-Octenylsuccinat, eignet sich damit hervorragend zur Trocknung, insbesondere Sprühtrocknung, von kosmetischen Zubereitungen, insbesondere von O/W-Emulsionen oder emulgatorfreien Hydrodispersionen.

Bevorzugt werden Celluloseether, insbesondere Hydroxypropylmethylcellulose (HPMC), aus Stärke gewonnene wasserlösliche Kohlenhydrate, insbesondere Maltodextrine, und nicht wasserlösliche modifizierte Stärken, insbesondere Aluminium-Stärke-Octenylsuccinat, im Verhältnis von ungefähr 0,5 bis 2 zu 0,5 bis 2 zu 0,5 bis 2, insbesondere im Verhältnis von 1 : 1 : 1 eingesetzt.

Eine O/W-Emulsion oder eine emulgatorfreie Hydrodispersion bzw. deren Konzentrate mit Hydroxypropylmethylcellulose, Maltodextrine, insbesondere MD 12, und Aluminium-Stärke-Octenylsuccinat bilden die erfindungsgemäß bevorzugten Ausführungsformen.

Ferner ist es erfindungsgemäß bevorzugt, wenn der Wassergehalt in dem Konzentrat nach der Trocknung, bevorzugt der Sprüh- und/oder Gefriertrocknung, weniger als 8 Gewichts-% und besonders bevorzugt weniger als 5 Gew.-%, jeweils bezogen auf das Gesamtgewicht der getrockneten Emulsion beträgt.

Vorteilhaft ist natürlich, dass sich die trockenen Zubereitungen bzw. Emulsionen, das Konzentrat, durch einfachen Zusatz von Wasser "reemulgieren" lassen, das heißt, dass wieder eine wasserhaltige Zubereitung bzw. O/W-Emulsion bzw. Hydrodispersion ausgebildet wird.

Die erfindungsgemäßen Zubereitungen weisen gegenüber den bisher bekannten, redispergierbaren Zubereitungen ein besonders angenehmes Hautgefühl auf und lassen sich mit einer Vielzahl kosmetischer Wirk-, Hilfs- und Zusatzstoffen kombinieren. So können die erfindungsgemäßen Emulsionen UV-Lichtschutzfilter enthalten, die sich bisher nicht in reemulgierbare Emulsionen einarbeiten ließen.

Die Ölphase der erfindungsgemäßen Emulsion, bevorzugt der O/W-Emulsion, d.h. die lipophilen organischen Bestandteile, werden vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isoprapylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Besonders bevorzugte Öle sind zu wählen aus der Gruppe
- Octyldodecanol
- Paraffinum Liquidum
- C13-16 Isoparaffin
- Isopropyl Palmitate
- Dicaprylylcarbonat
- Capryl/Caprin Triglyceride
- Butylenglykol Dicaprylat/Dicaprat
- Phenylbenzoat und/oder
- C12-C15 Alkyl Benzoate

In Kombination mit Lichtschutzfiltem sind die zuvor genannte Öle bevorzugt zu wählen, insbesondere in Kombination mit Butylenglykol Dicaprylat/Dicaprat, Phenylbenzoat und/oder C12-C15 Alkyl Benzoate.

Bevorzugt können die erfindungsgemäßen Zubereitungen einen geringen Lipidanteil aufweisen. Emulsionen mit geringerem Lipidanteil lassen sich generell besser trocknen, ergeben aber mitunter weit weniger reichhaltige Lotionen mit einer frischeren, wässrigeren Sensorik.
Geringer Lipidanteil bedeutet dabei ein Anteil an Lipiden von insgesamt ungefähr weniger als 10 Gew.%, bezogen auf die Gesamtmasse der O/W-Emulsion bzw. Hydrodispersion. Bevorzugt werden weniger als 10 Gew.% Paraffinum Liquidum, C13-16 isoparaffin und Isopropyl Palmitate eingesetzt.

Die erfindungsgemäße Zubereitung kann erfindungsgemäß ein oder mehrere Emulgatoren enthalten, wobei für die für kosmetische Emulsionen bekannten O/W-Emulgatoren eingesetzt werden können.

Ebenso sind emulgatorfreie Hydrodispersionen bevorzugte Zubereitungen, die sich erfindungsgemäß durch Sprühtrocknung zu einem Konzentrat trocknen lassen. HPMC zeigt emulgierende Eigenschaften und kann Emulsionen stabilisieren. Desweiteren können Verdicker, wie Carbomere, Acrylates/C10-30 Alkyl Acrylate Crosspolymere eingesetzt werden, die ebenfalls die Emulsion stabilisieren und den Einsatz von Emulgatoren überflüssig machen können. Die Inhaltsstoffe der erfindungsgemäßen Hydrodispersion entsprechen somit den erfindungsgemäßen Emulsionsrezepturen nur ohne Emulgator, wie z.B. Sorbitan Stearat.

Acrylates/C10-30 Alkyl Acrylate Crosspolymer, im Stand der Technik mitunter als Polymeremulgator bezeichnet, wird erfindungsgemäß als Verdicker bezeichnet und eigesetzt.

Ferner ist es erfindungsgemäß, wenn eine erfindungsgemäße Zubereitung, insbesondere auf Basis einer O/W-Emulsion oder Hydrodispersion wasserlösliche kosmetische und/oder dermatologische Wirk-, Hilfs- und/oder Zusatzstoffe und/oder Polyole, UV-Filter, Moisturizer, kosmetische Wirkstoffe umfasst.

Vorteilhaft ist es weiterhin, wenn die Zubereitungen keine Fettalkohole oder Wachsalkohole umfassen. Insbesondere keine linearen, gesättigten oder ungesättigten primären Alkohole *(Alkan-1-ole)* mit 6-22 Kohlenstoff-Atomen oder höhermolekulare Alkohole (Wachsalkohole).

Vorteilhaft im Sinne der vorliegenden Erfindung enthält das erfindungsgemäße Konzentrat auch einen oder mehrere Tablettenhilfsstoffe.

Erfindungsgemäß ist nicht zuletzt das Verfahren zur Herstellung eines Kosmetikums, welches dadurch gekennzeichnet ist, dass ein erfindungsgemäßes Konzentrat (eine durch Sprühtrocknung und/oder Gefriertrocknung getrocknete Zubereitung, insbesondere auf Basis einer O/W-Emulsion oder Hydrodispersion, die gegebenenfalls mit wasserlöslichen und/oder leichtflüchtigen Wirk-, Hilfs-, Zusatz- und/oder Tablettenhilfsstoffen versetzt sowie gegebenenfalls tablettiert bzw. granuliert wurde) mit Wasser versetzt bzw. in Wasser emulgiert wird. Erfindungsgemäß sind demnach Kosmetika, die nach diesem Verfahren hergestellt werden.

Ein derartiges erfindungsgemäßes Kosmetikum liegt dann vorteilhaft in Form einer Salbe, Creme, Lotion oder eines Emulsionsschaumes (franz. Mousse) oder einer sprühbaren Form vor. Es kann erfindungsgemäß vorteilhaft zur Behandlung und Pflege der Haut, Haare und Nägel eingesetzt werden. Erfindungsgemäß bevorzugt ist dabei die Verwendung als Sonnenschutzmittel, sofern insbesondere Lichtschutzfilterbestandteile enthalten sind.

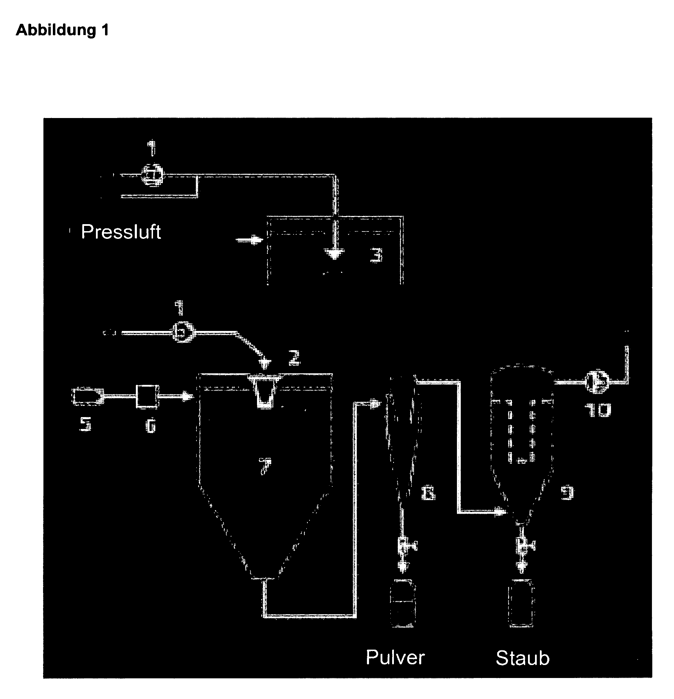

Der Verlauf einer Sprühtrocknung ist schematisch in Abbildung 1 dargestellt. Die Sprühtrocknung ist ein kontinuierlich durchführbares Verfahren zur Trocknung von Lösungen, Suspensionen oder pastösen Massen (1).

Mittels einer Düse (3), die durch Flüssigkeitsdruck oder Pressluft bzw. Inertgas betrieben werden kann, oder rotierenden Zerstäuberscheiben (2) (mit ca. 4000-50000 U/min) wird das zu trocknende Gut (1) in einen Heißluftstrom (5, 6) eingebracht. Die Temperatur variiert je nach Apparatur bis zu 220° C. Der Heißluftstrom trocknet das Gut in Bruchteilen einer Sekunde zu einem feinen Pulver. Die Heißluft kann in Richtung mit dem Sprühstrahl oder gegen den Sprühstrahl strömen (Gleichstrom-, Gegenstromverfahren), je nach Bauart oder Verwendungszweck.

Die Sprüheinrichtung befindet sich am oberen Teil eines Sprühturms (7), das anfallende Trockengut wird meist durch einen Zyklonabscheider (8) vom Luftstrom (9, 10) getrennt und kann dort entnommen werden.

Meist bilden sich im Konzentrat Hohlkugeln (20-200µm) mit großer spezifischer Oberfläche, welche für gewisse Produkte eine gute Löslichkeit gewährleistet.

Bekannte Beispiele für die Anwendung der Sprühtrocknung sind Milchpulver (Trockenmilch) und Pulverleim.

Die Sprühtrocknung dient auch als Verfahren zur Mikroverkapselung ätherischer Öle oder anderer oxidationsempfindliche Stoffe.

Zum Beleg der vorteilhaften Eigenschaften der erfindungsgemäßen Zubereitungen bzw. der Konzentrate wurden folgende Vergleichsversuche durchgeführt.
Ziel der Versuche war es, den Einfluss unterschiedlicher Trägermaterialien auf das erhaltene Konzentrat zu untersuchen.
Es wurden drei O/W-Emulsionen hergestellt, wie in Tabelle 1 angegeben.

Dabei enthält Zusammensetzung 103 die erfindungsgemäße Kombination aus Hydroxypropylmethylcellulose (HPMC), Maltodextrin (MD12) und Octenylsuccinat Stärke. Zubereitung 105 beinhaltete nur HPMC und Octenylsuccinat Stärke (kein Maltodextrin) und Zubereitung 106 beinhalteten nur Maltodextrin und Octenylsuccinat Stärke (kein HPMC).

**Tabelle 1: Vergleichsversuche**

| | | | |
|---|---|---|---|
| INCI | 103 | 105 | 106 |
| Aqua | 73,75 | 75,25 | 72,75 |
| Sorbitan Stearate | 1 | 1 | 1 |
| Trisodium EDTA | 0,1 | 0,1 | 0,1 |
| Butyl Methoxydibenzoylmethane | 1,5 | 1,5 | 1,5 |
| Äqua + Sodium Hydroxide | 0,1 | 0,1 | 0,1 |
| Aluminium Starch Octenylsuccinate + Aqua | 3 | 3 | 3 |
| Ethylhexyl Triazone | 0.5 | 0,5 | 0,5 |
| Titanium Dioxide + Trimethoxycaprylylsilane | 1,6 | 1,6 | 1,6 |
| HPMC | 3 | 4,5 | 0 |
| Acrylates/C10-30 Alkyl Acrylate Crosspolymer | 0,15 | 0,15 | 0,15 |
| Bis-Ethylhexyloxyphenol Methoxyphenyl Triazine | 1 | 1 | 1 |
| Diethylhexyl Butamido Triazone | 1,3 | 1,3 | 1,3 |
| DMDM Hydantoin | 0.3 | 0,3 | 0,3 |
| Phenethyl Benzoate | 9,7 | 9,7 | 7,7 |
| Maltodextrin (12) | 3 | 0 | 0 |
| Maltodextrin (19) | 0 | 0 | 9 |

| | | | |
|---|---|---|---|
| Öl + Feststoff [%] | 26,25 | 24,75 | 27,25 |
| Feststoffe [%] | 10,6 | 9,1 | 13,6 |
| Feststoffanteil des getrockneten Pulvers [%] | 40,38 | 36,77 | 49,91 |

### Trocknungsbedingungen

Kurz vor der Sprühtrocknung wurde den Emulsionen Wasser zugesetzt, um die benötigte Viskosität zu erreichen.

| | 103 | 105 | 106 |
|---|---|---|---|
| Emulsionsmenge [kg] | 2,14 | 2,07 | 2,10 |
| Wasserzusatz [kg] | 1,00 | 1,30 | 1,00 |
| Gesamtmenge [kg] | 3,14 | 3,37 | 3,10 |

Die Einlasstemperatur wurde bei allen drei Ansätzen auf ∼ 176°C belassen, die Auslasstemperatur lag bei ∼ 90°C.
Die reine Trocknungszeit betrug durchschnittlich 15 min.

| | 103 | 105 | 106 |
|---|---|---|---|
| Erwartete Pulvermenge [g] | 561,7 | 512,3 | 572,3 |
| Tatsächliche Pulvermenge [g] | 350 | 303 | 94 |
| Ausbeute [%] | 62,3 | 59,1 | 16,42 |

Die Kombination der erfindungsgemäßen Trägermaterialien des Ansatzes 103 zeigt eindeutig eine höhere Ausbeute. Durch einfaches Klopfen an der Sprühturmwand konnte das Pulver leicht von dieser abgelöst werden.
Bei den Ansätzen 105 und vor allem 106 war eine starke bis sehr starke Verkrustung der Sprühturmwand zu beobachten. Nur mit Hilfe eines Handfegers konnte bei 105 eine Ausbeute von 59% sichergestellt werden.
Mit einer Ausbeute von nur 16% kann Maltodextrin mit Aluminum Starch Octenylsuccinate als Trägermaterialien ausgeschlossen werden. Während der Trocknung bildete sich eine sehr feste Schicht an der Turmwand (106).

Auch die sensorischen Eigenschaften der erfindungsgemäßen Zubereitungen sind als äußerst zufrieden stellend zu beschreiben. Dies insbesondere auch mit und ohne Lichtfiltersubstanzen.

Als Trocknungsverfahren zur Herstellung eines erfindungsgemäßen Konzentrates einer O/W-Emulsion bzw, einer emulgatorfreien Hydrodispersion kann neben der bevorzugten Sprühtrocknung auch die Gefriertrocknung, die Kanaltrocknung oder die Walzentrocknung zur Anwendung kommen,
Unter Gefriertrocknung (Lyophilisation) versteht man eine Grundoperation der Aufarbeitungstechnik, bei der die flüssige Phase (in den meisten Fällen Wasser) durch Sublimation aus dem gefrorenen Gut unter Umgehung des flüssigen Aggregatzustandes entzogen wird.

Aus dem Phasendiagramm des Wassers ist ersichtlich, dass Sublimation nur unterhalb des Tripelpunktes erfolgen kann. Für die Gefriertrocknung ist daher die Temperatur wesentlich, bei der nur noch eine feste Phase vorliegt (eutektischer Bereich). Bei komplex zusammengesetzten Produkten liegt dieser Punkt meist viel tiefer, als bei Wasser und der Wasserdampfpartialdruck ist an der Sublimationsfläche sehr gering. Daher ist ein entsprechendes Vakuum für die Gefriertrocknung erforderlich. Während des Trocknungsvorganges darf die Temperatur des zu trocknenden Gutes diesen Wert nicht übersteigen, um ein Auftauen zu verhindern.

Die für die Sublimation benötigte Energie (2845 kJ/kg Wasser) muss während des Trocknungsvorgangs von außen an die Sublimationszone herangeführt werden. Dies geschieht durch Kontakt, durch Strahlung und in Abhängigkeit vom Vakuum in der Trockenkammer, durch Konvektion. Da unterhalb von 10⁻² mbar praktisch keine Wärmeübertragung mehr durch Konvektion möglich ist, muss das Vakuum stets so schlecht wie möglich gehalten werden, aber noch gut genug, um ein Auftauen zu verhindern. Die Druckregulierung muss daher bei der Gefriertrocknung sehr genau erfolgen.

Die Gefriertrocknung ist sicher die schonenste Trocknung und wird bei besonders temperaturempfindlichen Bioprodukten angewendet. Überwiegend erfolgt die Gefriertrocknung in Vakuumtunnel- oder Vakuumteller-Trocknem innerhalb von 1 bis 3 Stunden. Die Vorteile der Gefriertrocknung liegen in den niedrigen Temperaturen, bei denen keine chemische Veränderung des Produktes auftritt. Der technische Aufwand (Erzeugung des Vakuums und der tiefen Temperatur von -45 °C im Kondensator) und der spezifische Energiebedarf sind jedoch hoch, so dass die Anwendung bisher auf kostenintensive Produkte, wie Hormon-, Enzym- und Vitaminpräparate, Plasma- und Serumkonserven zur Bluttransfusion, Viren, Bakterien (Lebendimpfstoffe, Stammhaltung), angewendet wurde.

Bei der Kanaltrocknung passiert das ausgebreitete Nassgut, mechanisch fortbewegt (Transportband), einen Kanal, der durch Dampf, Heißwasser oder Heißluft beheizt ist. Ventilatoren sorgen für eine Luftumwälzung. Das Trockengut wird am Kanalende entnommen. Auch Trockentrommeln und solche Einrichtungen, bei denen das Trocknungsmaterial durch Rührer oder andere Einbauten bewegt wird, z.B. Schnecken-, Schaufel- oder Muldentrockner sind vorteilhaft. Sie werden gleichfalls beheizt und gestatten einen kontinuierlichen Materialfluss,

Walzentrockner bewähren sich zum Entfernen der Flüssigkeit aus viskosem Material. Aus einem Vorratsbehälter wird die Lösung mit Hilfe einer Auftragswalze in dünner Schicht auf eine von innen beheizte Metallwalze aufgebracht und es erfolgt eine Kontakttrocknung. Der Trocknungsprozess dauert nur einige Sekunden, denn nach einer knappen Umdrehung wird das getrocknete Material mit Schabern von der Walze abgenommen. Substanzen, die selbst dieser kurzfristigen Erhitzung nicht standhalten, lassen sich auf Vakuumwalzentrocknem entwässern.

Durch Trocknung, wie zuvor beschriebene Sprüh-, Gefrier-, Kanal- oder Walzentrocknung, einer O/W-Emulsion bzw. emulgatorfreien Hydrodispersion bildet sich ein erfindungsgemäßes Konzentrat.
Nach einer erneuten Rehydration des Konzentrates ergibt sich die erfindungsgemäße kosmetische Zubereitung, bevorzugt in Form einer Salbe, Creme, Lotion oder eines Emulsionsschaumes (franz. Mousse) oder einer sprühbaren Form vor. Das Konzentrat kann daher platz- und kostensparend transportiert und gelagert werden und erst vor Anwendung mit Wasser versetzt oder aber auch pur zur Anwendung kommen. Es kann erfindungsgemäß vorteilhaft zur Behandlung und Pflege der Haut, Haare und Nägel eingesetzt werden. Erfindungsgemäß bevorzugt ist dabei die Verwendung als Sonnenschutzmittel.

Es ist auch erfindungsgemäß, die getrocknete Emulsion bzw. Hydrodispersion, also das Konzentrat per se, ohne weiteren Wasserzusatz, also ohne Redispergierung, direkt auf die Haut aufzutragen. Dies kann in Form von z.B. "Körper-Puder" oder Stiften, insbesondere Puder- und/oder Abdeck-Stiften, geschehen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiel 1: O/W Emulsion ohne Lichtfilter für eine Anwendung als Bodylotion

| | Gew.% |
|---|---|
| Methylparaben | 0,3 |
| Isopropyl Palmitate | 5 |
| Aqua | 74,95 |
| Sorbitan Stearate | 0,5 |
| Paraffinum Liquidum | 5 |
| Aqua + Sodium Hydroxide | 0,1 |
| Aluminum Starch Octenylsuccinate + Aqua | 3 |
| Hydroxypropylmethylcellulose | 3 |
| Acrylates/C10-30 Alkyl Acrylate Crosspolymer | 0,15 |
| C13-16 Isoparaffin | 5 |
| Maltodextrin (MD12) | 3 |

### Beispiel 2: O/W Emulsion ohne Lichtfilter für eine Anwendung als Bodylotion (geringerer Lipidanteil)

| | Gew.% |
|---|---|
| Methylparaben | 0,3 |
| Isopropyl Palmitate | 3 |
| Aqua | 80,95 |
| Sorbitan Stearate | 0,5 |
| Paraffinum Liquidum | 3 |
| Aqua + Sodium Hydroxide | 0,1 |
| Aluminum Starch Octenylsuccinate + Aqua | 3 |
| Hydroxypropylmethylcellulose | 3 |
| Acrylates/C10-30 Alkyl Acrylate Crosspolymer | 0,15 |
| C13-16 Isoparaffin | 3 |
| Maltodextrin (MD12) | 3 |

### Beispiel 3: Hydrodispersion ohne Lichtfilter für eine Anwendung als Bodylotion

| | Gew.% |
|---|---|
| Methylparaben | 0,3 |
| Isopropyl Palmitate | 3 |
| Aqua | 81,45 |
| Paraffinum Liquidum | 3 |
| Aqua + Sodium Hydroxide | 0,1 |
| Aluminum Starch Octenylsuccinate + Aqua | 3 |
| Hydroxypropylmethylcellulose | 3 |
| Acrylates/C10-30 Alkyl Acrylate | 0,15 |
| Crosspolymer | |
| C13-16 Isoparaffin | 3 |
| Maltodextrin (MD12) | 3 |

### Beispiel 4: O/W Emulsion mit Lichtfiltern

| | Gew.% |
|---|---|
| Aqua | 73,75 |
| Sorbitan Stearate | 1 |
| Trisodium EDTA | 0,1 |
| Butyl Methoxydibenzoylmethane | 1,5 |
| Aqua + Sodium Hydroxide | 0,1 |
| Aluminum Starch Octenylsuccinate + Aqua | 3 |
| Ethylhexyl Triazone | 0,5 |
| Titanium Dioxide + Trimethoxycaprylylsilane | 1,6 |
| Hydroxypropylmethylcellulose | 3 |
| Acrylates/C10-30 Alkyl Acrylate Crosspolymer | 0,15 |
| Bis-Ethylhexyloxyphenol Methoxyphenyl Triazine | 1 |
| Diethylhexyl Butamido Triazone | 1,3 |
| DMDM Hydantoin | 0,3 |
| Phenethyl Benzoate | 9,7 |
| Maltodextrin (MD12) | 3 |

### Bezugszeichenliste zur Abbildung 1

- 1.: Pumpe (Masse)
- 2.: rotierende Zerstäuberscheibe
- 3.: Düse
- 5.: Filter
- 6.: elektrische Heizquelle
- 7.: Sprühturm
- 8.: Zyklonabscheider
- 9.: Filter
- 10.: Abluftgebläse

## Patentansprüche

1. Kosmetische Zubereitung, insbesondere auf Basis einer O/W Emulsion oder einer emulgatorfreien Hydrodispersion, umfassend ein oder mehrere Celluloseether, ein oder mehrere aus Stärke gewonnene wasserlösliche Kohlenhydrate und ein oder mehrere nicht wasserlösliche modifizierte Stärken.

2. Konzentrat erhältlich durch Sprüh-, Gefrier-, Kanal- und/oder Walzentrocknung, insbesondere durch Sprühtrocknung, einer Zubereitung nach Anspruch 1.

3. Zubereitung oder Konzentrat nach einem der vorstehenden Ansprüche, **dadurch gekennzeichnet, dass** als Celluloseether Hydroxypropylmethylcellulosen, Methylhydroxyethylcellulosen, Methylhydroxypropylcellulosen, Methylhydroxybutylcellulosen, Ethylhydroxyethylcellulosen, Carboxymethylhydroxyethylcellulosen, Carboxymethylcellulosen, Methylcellulosen, Hydroxyethylcellulosen, Carboxymethylhydroxyethylcelulosen, Ethylcellulosen, Ethylhydroxyethylcellulosen und/oder Hydroxypropylcellulosen, insbesondere Hydroxypropylmethylcellulose (HPMC), gewählt werden.

4. Zubereitung oder Konzentrat nach einem der vorstehenden Ansprüche, **dadurch gekennzeichnet, dass** als aus Stärke gewonnene wasserlösliche Kohlenhydrate Stärkehydrolysate, insbesondere Maltose, Maltit, Glucosesirup, Maltodextrine, Cyclodextrine, Dextrose, Trockenglucosesirup, Sorbit und/oder Fructose, insbesondere Maltodextrine, gewählt werden.

5. Zubereitung oder Konzentrat nach einem der vorstehenden Ansprüche, **dadurch gekennzeichnet, dass** als nicht wasserlösliche modifizierte Stärke Stärkeester und/oder - ether, insbesondere Succinate, Xanthogenate, Acetate, Phosphate, Sulfate und/oder Nitrate, insbesondere Aluminium-Stärke-Octenylsuccinat, gewählt werden.

6. Zubereitung oder Konzentrat nach einem der vorstehenden Ansprüche, **dadurch gekennzeichnet, dass** der oder die Celluloseether, ein oder mehrere aus Stärke gewonnene wasserlösliche Kohlenhydrate und eine oder mehrere nicht wasserlösliche modifizierte Stärken im Verhältnis von 0,5 bis 2 zu 0,5 bis 2 zu 0,5 bis 2, insbesondere im Verhältnis von 1 zu 1 zu 1, gewählt werden.

7. Zubereitung oder Konzentrat nach einem der vorstehenden Ansprüche umfassend ein oder mehrere Lichtschutzfiltersubstanzen.

8. Zubereitung oder Konzentrat nach einem der vorstehenden Ansprüche umfassend ein oder mehrere Öle gewählt aus Octyldodecanol, Paraffinum Liquidum, C13-16 Isoparaffin, Isopropyl Palmitate, Dicaprylylcarbonat, Capryl/Caprin Triglyceride, Butylenglykol Dicaprylat/Dicaprat, Phenylbenzoat und/oder C12-C15 Alkyl Benzoate, insbesondere Butylenglykol Dicaprylat/Dicaprat, Phenylbenzoat und/oder C12-C15 Alkyl Benzoate.

9. Zubereitung oder Konzentrat nach einem der vorstehenden Ansprüche umfassend keine Fett- und/oder Wachsalkohole.

10. Verwendung der Kombination aus ein oder mehreren Celluloseethern, insbesondere Hydroxypropylmethylcellulose (HPMC), ein oder mehreren aus Stärke gewonnenen wasserlöslichen Kohlenhydraten, insbesondere Maltodextrine, und ein oder mehreren nicht wasserlöslichen modifizierten Stärken, insbesondere Aluminium-Stärke-Octenylsuccinat, zur Trocknung, insbesondere Sprühtrocknung, von kosmetischen Zubereitungen, insbesondere O/W-Emulsionen oder emulgatorfreien Hydrodispersionen.

11. Verwendung nach Anspruch 1, **dadurch gekennzeichnet, dass** der oder die Celluloseether, ein oder mehrere aus Stärke gewonnene wasserlösliche Kohlenhydrate und eine oder mehrere nicht wasserlösliche modifizierte Stärken im Verhältnis von 0,5 bis 2 zu 0,5 bis 2 zu 0,5 bis 2, insbesondere im Verhältnis von 1 zu 1 zu 1, gewählt werden.
